# EUROPÄISCHE PATENTSCHRIFT

(11) **EP 1 913 394 B1**
(45) Veröffentlichungstag und Bekanntmachung des Hinweises auf die Patenterteilung: **20.01.2010**
(21) Anmeldenummer: 06775810.2
(22) Anmeldetag: 04.08.2006
(51) Int. Cl.: G01N 33/569

(54) **TESTSYSTEM ZUM NACHWEIS VON SALMONELLEN**
TEST SYSTEM FOR DETECTING SALMONELLA
SYSTÈME DE TEST SERVANT AU DÉPISTAGE DE SALMONELLES

(30) Priorität: 07.08.2005 DE 102005037796
(43) Veröffentlichungstag der Anmeldung: 23.04.2008
(73) Patentinhaber: TGC Biomics GmbH, 55444 Schweppenhausen (DE)
(72) Erfinder: VON EICHEL-STREIBER, Christoph, 55444 Schweppenhausen (DE); KLEIN, Harald, 64546 Mörfelden (DE)
(74) Vertreter: Rudolph, Ulrike
(86) Internationale Anmeldenummer: PCT/DE2006/001372
(87) Internationale Veröffentlichungsnummer: WO 2007/016912

(56) Entgegenhaltungen:
- WO-A-00/23462
- WO-A-97/18225
- EICHELBERG KATRIN ET AL: "The flagellar sigma factor fliA (sigma28) regulates the expression of Salmonella genes associated with the centisome 63 type III secretion system" INFECTION AND IMMUNITY, Bd. 68, Nr. 5, Mai 2000 (2000-05), Seiten 2735-2743, XP002405150 ISSN: 0019-9567
- MUSSON JULIE A ET AL: "Processing of viable Salmonella typhimurium for presentation of a CD4 T cell epitope from the Salmonella invasion protein C (SipC)" EUROPEAN JOURNAL OF IMMUNOLOGY, Bd. 32, Nr. 9, September 2002 (2002-09), Seiten 2664-2671, XP002405151 ISSN: 0014-2980
- FANG ET AL.: "Identification of Salmonella using colony-print and detection with antibody-coated gold nanoparticles" JOURNAL OF MICROBIOLOGICAL METHODS, 2009, pages 1-4,
- WILSON, G.: "Rapid and economical method for biochemical screening of stool isolates for salmonella and shigella species" JOURNAL OF CLINICAL MICROBIOLOGY, vol. 42, no. 10, October 2004 (2004-10), pages 4821-4823,
- RUSHDY, A.A. ET AL.: "Application of molecular methods to a nosocomial outbreak of salmonella enteridis phage type 4" JOURNAL OF HOSPITAL INFECTION, vol. 36, 1997, pages 123-131,
- GOPAL RAO, Q. ET AL: "Rational protocols for testing faeces in the investigation of sporadic hospital-acquired diarrhoea." JOURNAL OF HOPITAL INFECTION, vol. 47, 2001, pages 79-83,
- Salmonella rapid detection interagency group meeting - executive summary.
- FDA seeks rapid test for salmonella
- AILSA CAMPBELL: "Encyclopedia of immunology - Volume 3 - Monoclonal Antibodies" 1992, ACADEMIC PRESS LIMITED , LONDON
- KOMORIYA KAORU ET AL: "Flagellar proteins and type III-exported virulence factors are the predominant proteins secreted into the culture media of Salmonella typhimurium" MOLECULAR MICROBIOLOGY, vol. 34, no. 4, November 1999 (1999-11), pages 767-779,
- WATSON P.R. ET AL: "Mutation of invH, but not stn, reduces Salmonella-induced enteritis in cattle." INFECTION AND IMMUNITY, vol. 66, no. 4, April 1998 (1998-04), pages 1432-1438,
- COLLAZO C.M.; GALAN J.E.: "The invasion-associated type III system of Salmonella typhimurium directs the translocation of Sip proteins into the host cell" MOLECULAR MICROBIOLOGY, vol. 24, no. 4, 1997, pages 747-756,

## Beschreibung

Die Erfindung betrifft ein Testsystem zum Nachweis von Salmonelleninfektionen in einer Probe.

Salmonellen sind bewegliche, gramnegative Stäbchenbakterien. Die taxonomische Unterscheidung der Salmonellen erfolgt anhand ihrer Körper(O)- und Geißel(H)-Antigene nach dem Kauffmann-White-Schema (einer diagnostischen Antigentabelle) und resultiert in einem Ordnungssystem, in dem die Salmonellen als Serovare deklariert und anhand einer Seroformel charakterisiert und klassifiziert sind. Von den bisher bekannten über 2400 Salmonella-Serovaren haben in der Praxis bisher nur zwanzig bis dreißig eine Bedeutung als Erreger von epidemiologischen Erkrankungen. Hierzu gehören die Erreger des Typhus (*S*. *typhi)* und Paratyphus (*S. paratyphi A, S. paratyphi B, S. paratyphi C*) und eine große Anzahl von Enteritis-Erregern, die sogenannten "Enteritis-Salmonellen". Während die Typhus- und Paratyphus-Salmonellen schwere, generalisierte Allgemeininfektionen, das heißt Infektionen des ganzen Körpers hervorrufen, ist die Infektion mit Enteritis-Sahnonellen in der Regel lokal auf den Darm beschränkt.

Salmonellosen des Menschen sind zumeist lebensmittelbedingte Erkrankungen. Die Infektion erfolgt in der Regel durch den Verzehr infizierter oder kontaminierter Lebensmittel, wogegen die Übertragung auf den Menschen durch den direkten Kontakt mit salmonellenausscheidenden Tieren nur selten erfolgt. Eine direkte oder indirekte Übertragung von Mensch zu Mensch kann als Hospitalinfektion bei besonders disponierten Personen oder unter hygienisch ungünstigen Bedingungen erfolgen.
Primäre Infektionsquellen sind besonders von Geflügel, Rindern und Schweinen stammende Lebensmittel, wobei die Tiere in den seltensten Fällen selbst erkrankt sind.
Demzufolge kommt dem Erreger- bzw. Antikörper-Nachweis nicht nur in der Humanmedizin, sondern auch in der Veterinärmedizin und in Lebensmittelbetrieben eine entscheidende Bedeutung zu. Die Infektionsdosis für den erwachsenen Menschen liegt bei 10⁴ bis 10⁶ Keimen. Die Inkubationszeit beträgt 5-72h und ist abhängig von der Höhe der Infektionsdosis.

Im Fall einer Enteritis-Salmonellen-Infektion, der sogenannten Enteritis-Salmonellose, äußert sich die Infektion zunächst mit Durchfall, Brechreiz oder Erbrechen und mäßigem Fieber. Die Symptome dauern in der Regel nur wenige Stunden oder Tage an. Bei geschwächten Patienten kann jedoch auch diese Erkrankung zum Tod führen.
Die Keim-Ausscheidung von Enteritis-Salmonellen dauert im Mittel drei bis sechs Wochen, bei Säuglingen aber auch über Monate.

Bei erblich vorbelasteten Patienten können die Salmonellen als Zweiterkrankung 2-6 Wochen nach dem enteritischen Infekt eine reaktive Arthritis auslösen, welche in seltenen Fällen eine chronische Verlaufsform annimmt. Da zu diesem Zeitpunkt meist keine Erreger mehr nachweisbar bzw. anzüchtbar sind, haben serologische Verfahren zum Nachweis einer zeitlich zurückliegenden (= in der Vergangenheit bestandenen) Salmonelleninfektion große Bedeutung (Widal-Agglutination; s. u.).

Gemäß Infektionsschutzgesetz ist der Verdacht auf oder die Erkrankung an akuter infektiöser Gastroenteritis unter bestimmten Umständen meldepflichtig, ebenso jeglicher Nachweis von Salmonellen. Desweiteren befassen sich in Deutschland und anderen EU-Ländern verschiedenen Rechtsvorschriften mit den Maßnahmen zur Salmonellenbekämpfung (z.B.: Rinder-Salmonellen-Verordnung, Hühner-Salmonellen-Verordnung, verschiedenen Bestimmungen des Futtermittel- und Lebensinittelrechtes). Darüber hinaus sind im Rahmen der betrieblichen Eigenkontrolle verschiedene Salmonellenmonitoringprogramme etabliert worden.

Der Schwerpunkt der klinischen Labordiagnostik der Salmonellosen liegt in der Anzucht der Erreger aus Stuhlproben und ihrer Einordnung als Salmonellen-Verdachtsfälle mit Hilfe von omni- bzw. polyvalenten Salmonellen-Suchseren. In der Regel kann eine solche Verdachtsdiagnose erst ca. ein bis zwei Tage nach Eingang der Probe im diagnostischen Labor gestellt werden. Für die gesicherte Aussage, daß eine Salmonellen-Infektion vorliegt, werden normalerweise weitere 2-3 Tage benötigt. In dieser Zeit werden verdächtige Einzelklone biochemisch (bunte Reihe) und serologisch charakterisiert. Für die serologische Differenzierung werden die O- und H-Antigene in Form einer Objektträger-Agglutination (Kaufmann-White Schema) untersucht. Dazu finden zunächst polyvalente Salmonellen-Testseren Anwendung, um nachfolgend mit monovalenten O-bzw. H-Antiseren die genaue Antigenformel zu ermitteln. In der Regel werden daher für den definitiven Nachweis einer Salmonellen-Infektion insgesamt 3-5 Tage benötigt. Insbesondere bei Salmonellosen, die durch kontaminierte Lebensmittel hervorgerufen wurden, stellt der lange Zeitraum bis zur Diagnose ein großes Problem dar, weil sich zwischenzeitlich weitere Personen infizieren können. Es kommt deshalb bei einem Salmonellenverdacht darauf an, die Infektionsquelle zu lokalisieren und damit die weitere Verbreitung zu verhindern. Daneben werden zusätzlich die Patienten isoliert, um die Übertragung der Infektion zu vermeiden. Für den Erfolg dieser Maßnahme ist eine frühe Diagnose-Stellung von großer Bedeutung.

Der serologische Antikörpernachweis gegen Salmonellen spielt vor allem in der Veterinärmedizin und Lebensmittelindustrie in Form von ELISA-Systemen eine grosse Rolle. So wird z. B. in Deutschland wie auch in angrenzenden Nachbarländern derzeit vor allem der Salmonellen-Antikörperstatus (vorwiegend Anti-LPS-Immunglobulin) von Tierbeständen ermittelt. Der Nachweis erfolgt aus/in Fleischsaft oder Blut. Allerdings weist dieses Verfahren Einschränkungen auf, da es nicht alle pathogenen Salmonellen-Serovare zuverlässig nachweisen kann.

In der Humanmedizin wird dagegen insbesondere bei typhösen Salmonellen-Infektionen der sog. Widal-Agglutinationstest als Ergänzung zum bakteriologischen Erregernachweis eingesetzt. Dabei wird Patientenserum mit abgekochten (O-Antigen-Agglutination) oder formalinisierten (H-Antigen-Agglutination) Salmonellensuspensionen versetzt und auf eine Agglutination der Bakterien untersucht. Nachteilig hierbei ist zum einen die Tatsache, dass nicht alle Infektionen mit der Ausbildung eines Anti-O-Antigen-Titers einhergehen, zum anderen dass Antikörper gegen H-Antigene über Jahre nach der Infektion persistieren können. Titer gegen O-Antigene fallen hingegen meist nach wenigen Wochen wieder ab. Somit kann nicht zuverlässig zwischen einer akuten oder bereits überwundenen Infektion unterschieden werden.

Unter in-vitro-Kulturbedingungen werden von den Salmonellen unterschiedliche Proteine in das umgebende Medium abgegeben. Eines dieser Protein ist das SipC-Protein.
Die Nukleotidsequenz des SipC-Gens ist bekannt und steht dem Fachmann über Gen-Datenbanken zur Verfugung (beispielsweise Accession No.: U25631 oder X82670).
Aus der WO 03/000935 ist die Verwendung von PCR-Primern und FRET-Hybridisierungssonden gegen das sipC-Gen zum Nachweis von Salmonellen bekannt. Der Einsatz des SicC-Proteins zu demselben Zweck, insbesondere der Einsatz von Antikörpern gegen das SipC-Protein, wird in dieser Druckschrift nicht angedacht.
Aus den Druckschrift Weinrauch et al. "Neutrophil elastase targets virulence factors of enterobacteria." (Nature 2002, 417, 6884, 91-4) und Hayward et al. "Direct nucleation and bunbdling of actin by the SipC protein of invasive Salmonella" (EMBO J. 1999, 18, 18, 4926-34) ist die Verwendung eines polyklonalen Antikörpers gegen das SipC-Protein zu dessen Nachweis im Immunoblot beschrieben.
Die beschriebenen polykonalen Antikörper sind jedoch nicht monospezifisch für Salmonellen und daher nicht für einen spezifischen Salmonellen-Nachweis - wie er vor allem in der Human-Diagnostik und Lebensmittelprüfung erforderlich ist - geeignet.

In der WO 97/18225 A ist die Nukleotidsequenz der Ssp (=Sip)-Gene SipA, SipB, SipC und SipD und die Aminosäuresequenz der davon kodierten Proteine SspA, SspB, SspC und SspD beschrieben. Es wird die allgemeine Aussage getroffen, dass Ssp(=Sip)-Proteine zur Herstellung, von Antikörpern eingesetzt werden können, und dass solche Antikörper als therapeutisches oder diagnostisches Agent verwendet werden können. Weitere Angaben, wie z.B. welches dieser Ssp-Proteine vorrangig zur Gewinnung von Antikörpern geeignet ist, sind nicht offenbart. Die Autoren weisen allerdings ausdrücklich darauf hin, dass im Überstand von Salmonellen Wildtyp-Kulturen eine große Anzahl von Proteinen vorhanden ist, und dass die Ssp-/Sip-Proteine eine starke Homologie mit den Shigella flexneri secreted proteins IpaA, IpaB, IpaC und IpaD aufweisen.

Im Zuge der vorliegenden Erfindung wurde überraschenderweise gefunden, (1.) daß es sich bei dem SipC-Protein um ein sehr konserviertes Molekül handelt, das heißt die Aminosäure-Sequenzen verschiedener Salmonellen-Serovare weisen nur geringe Unterschiede auf, und (2.) dass Salmonellen bereits zu einem sehr frühen Zeitpunkt die Proteine des TypIII-Sekretionssystems bilden.

In den Druckschriften Eichelberg et al. (Infection and Immunity, 2000, Bd. 68, Nr.5, 2735-2743), Collazo et al. (Molecular Microbiology 1997, Bd. 24, Nr. 4, 747-756) und Watson et al (Infection and Immunity, 1998, Bd. 66, Nr.4, 1432-1438) ist jeweils ein monoklonaler Antikörper genen SipC zwar erwähnt jedoch enthält keine dieser Druckschriften diejenigen Angaben (z.B. wo er hinterlegt ist, wie er generiert wurde, welche Spezifität er aufweist und wie sensitiv er reagiert), die es dem Fachmann ermöglichen den erwähnten anti-SipC-Antikörper zu erwerben oder nachzuarbeiten. Für die Fachwelt sind diese in den Druckschriften erwähnten Anti-SipC-Antikömer nicht nacharbeitbar nicht erwerbbar und somit praktisch nicht vorhanden.

Aufgabe der vorliegenden Erfindung ist die Bereitstellung eines monoklonalen Antikörpers gegen ein Salmonellen-Sip-Protein, der einen schnellen (wenige Stunden und nicht mehrere Tage dauernden) sensitiven (auch geringe Mengen erfassenden) und spezifischen (kreuzreaktionsfreien oder wenigstens -armen) Nachweis von Salmonellen-Infektionen erlaubt, und damit die Bereitstellung eines Nachweissystems für Salmonellen, das auf die Frage, ob bei einer bestimmten Probe eine Infektion mit Salmonellen, gegenwärtig vorliegt oder in der Vergangenheit vorlag, eine schnelle und zuverlässige Ja-oder Nein-Antwort liefert.

Eine Lösung der genannten Aufgabe besteht in der Bereitstellung eines Testsystems zum Nachweis von gegenwärtig und/oder in der Vergangenheit vorhandenen Salmonellen-Infektionen in einer Probe, dadurch gekennzeichnet, dass das Testsystem wenigstens einen monoklonalen Antikörper umfasst, der spezifisch mit dem Salmonellen-SipC-Protein reagiert, und der (a) von den Zellen der bei der DSMZ-Deutsche Sammlung von Mikroorganismen und Zellkulturen GmbH, Deutschland, am 01. Juni 2006 unter der Nummer DSM ACC2790 hinterlegten Hybridoma-Zellkultur mit der Laborbezeichung I5B2 erzeugt wird, oder der (b) von den Zellen der bei der DSMZ-Deutsche Sammlung von Mikroorganismen und Zellkulturen GmbH, Deutschland, am 01. Juni 2006 unter der Nummer DSM ACC2789 hinterlegten Hybridoma-Zellkultur mit der Laborbezeichung V1H7 erzeugt wird.

In der diagnostischen Anwendung hat das hier vorgeschlagene Testsystem aufgrund der spezifischen monoklonalen Antikörper vor allem den Vorteil, daß es die verschiedensten Salmonellen unabhängig vom jeweiligen Serovar-Typ erkennt und folglich einen Serovar-übergreifenden Salmonellen-Nachweis ermöglicht.
Dies ist mit den herkömmlichen Verfahren nicht möglich, weil diese Verfahren auf der Detektion von O- und H-Antigenen basieren, die bei den einzelnen Salmonellen-Serovaren in hoher Variabilität und unterschiedlichsten Kombinationen auftreten können. Ein weiterer Vorteil dieses erfindungsgemäßen Testsystems besteht darin, daß der Salmonellennachweis bereits wenige Stunden nach der Salmonellen Infektion bzw. nach dem Auftreten der ersten Symptome erfolgen kann, und zwar mit zuverlässigem Ergebnis, so daß schon sehr früh Maßnahmen zur Eindämmung der Salmonellen ergriffen werden können.

Bei dem vorgeschlagenen Testsystem handelt es sich vorzugsweise um einen ELISA (enzyme linked immunosorbent assay). Mit den hier erstmals beschriebenen monoklonalen Antikörpern I5B2 (erzeugt von der Hybridomazellkultur DSM ACC2790) und V1H7 (erzeugt von der Hybridomazellkultur DSM ACC2789) ist es erstmals gelungen, einen ELISA zu etablieren, der spezifisch mit Salmonellen reagiert und keine Kreuzreaktionen mit Salmonellen -verwandten Bakterien zeigt, insbesondere nicht mit Shigellen oder enteropathogenen E.coli.

Eine Lösung der genannten Aufgabe besteht auch in der Bereitstellung eines Testsystems zum Nachweis von Antikörpern gegen Salmonellen in einer Probe, das als Reagenz rekombinant hergestelltes oder natives, aus Salmonellen isoliertes SipC-Protein umfaßt.

Der Vorteil dieses Testsystems gegenüber dem herkömmlichen Antikörper-Nachweis in der Humanmedizin mittels der Widal-Agglutination besteht zum einen darin, daß Immunglobuline gegen ein serovarübergreifend konserviertes Salmonellen-Antigen, nämlich das SipC-Protein, detektiert werden und damit eine Steigerung in der Sensitivität des Testes erreicht wird. Hinzu kommt, dass das Testsystem, insbesondere in der Ausführungsform als ELISA, objektive Meßergebnisse liefert, und die Ergebnisse daher nicht mehr vom subjektiven Auge des Betrachters abhängig sind, wie es beim Ablesen der Agglutinationsreaktion der Fall ist. Im Gegensatz zur Widal-Reaktion, deren Durchführung bis zu ca. 18 h in Anspruch nehmen kann, liefert ein ELISA bereits nach wenigen Stunden das Testergebnis.

Zusätzlich kann das Testsystem zum Nachweis eines Anti-SipC-Titers in Fleisch, Serum oder anderen Körperflüssigkeiten von Nutztieren, sowie in Eiern verwendet werden. Beispielhaft sei hier die Verwendung des Fleischsaftes von Nutztieren als Probe erwähnt. Dabei wird der Fleischsaft mit dem Fachmann bekannten und geläufigen Methoden gewonnen und zum Beispiel im ELISA mit angelagertem SipC-Protein als Fängermolekül auf Anti-SipC-Antikörper untersucht.

Als Testsysteme im Sinne der vorliegenden Erfindung kommen insbesondere ELISA, Sandwich-ELISA, Western-Blot, Latex-Agglutionation, Line Blot und weitere dem Fachmann bekannte Methoden in Betracht.

Die Erfindung wird im folgenden anhand von Ausführungsbeispielen und Figuren näher erläutert.

Die Figuren zeigen:
- Fig. 1:: Western Blot-Analyse von Kulturüberständen verschiedener Salmonella-Serovare mit dem polyklonalen Antiserum gegen SipC. Die von den Bakterien in den Kulturüberstand sezernierten Proteine wurden nach TCA-Fällung über ein 10%iges (Figure 1A) bzw. 12,5%iges (Figure 1B) SDS-Gel aufgetrennt und anschliessend auf PVDF-Membranen übertragen. Der immunologische Nachweis erfolgte mit dem polyklonalen SipC-Antiserum (1:10000) und einem alkalische Phosphatase-markiertem Anti-Kaninchen-Antikörper (Dianova, 1:30000).
Molekulargewichtsstandards sind jeweils auf der linken Bildseite angegeben.
Figur 1A: Spur 1 = Salmonella hadar, Spur 2 = S. brandenburg,
Spur 3 = S. goldcoast, Spur 4 = S. senftenberg, Spur 5 = S. virchow
Figur 1B: Spur 1 = S. typhimurium, Spur 2 = S. enteritidis, Spur 3 = S. infantis,
Spur 4 = S. bovis morbificans
- Fig. 2:: Western Blot Streifen nach Reaktion des polyklonalen SipC-Antiserums (A), des monoklonalen Antikörpers I5B2 (B) und des monoklonalen Antikörpers V1H7 (C) mit rekombinantem Salmonellen-SipC.Protein (Spur 2) und rekombinantem Shigellen-IpaC-Protein (Spur 1).
- Fig. 3:: Western Blot-Streifen nach Reaktion von Teilfragmenten des Salmonellen-SipC-Antigens mit den monoklonalen Antikörpern I5B2 (A) und V1H7 (B). In Spur 1 ist die C-terminale Region von SipC (Aminosäuren 252-410) und in Spur 2 die N-terminale Region von SipC (Aminosäuren 1-200) aufgetragen.
- Fig. 4:: Nachweis des SipC-Proteins in diversen Salmonellenkultur-Überständen mittels ELISA.
- Fig. 5:: Nachweis des SipC-Proteins in diversen Salmonellenkultur-Überständen mittels Western Blot. M = Molekulargewichtsmarker, 1 = Überstand einer Kultur von Salmonella brandenburg, 2 = Überstand einer Kultur von S. typhi, 3 = Überstand einer Kultur von S. typhimurium, 4 = Überstand einer Kultur von S. enteritidis, 5 = Überstand einer Kultur von S. infantis, 6 = Überstand einer Kultur von S. bovismorbificans, 7 = Überstand einer Kultur von S. paratyphi B, 8 = Überstand einer Kultur von S. virchow, 9 = Überstand einer Kultur von S. hadar, 10 = Überstand einer Kultur von S. goldcoast
- Fig. 6:: Zeitabhängiger immunologischer Nachweis (ELISA) des SipC-Antigens in Kulturüberständen von Salmonella enteritidis und S. typhimurium. Negativkontrolle = Kulturmedium; Positivkontrolle = 50ng und 5ng abs. rekombinantes SipC.
- Fig. 7:: Titration der Seren (nach der 1. Boosterimmunisierung) von mit rekombinantem SipC-Protein immunisierten Mäusen; Negativkontrolle = gepooltes Präimmunserum

Alle in den folgenden Beispielen genannten Verfahren sind dem Fachmann bekannt und sind z.B. in Ausubel et al. (2003) beschrieben.

### Beispiel 1: Herstellung von monoklonalen Antikörpern, die spezifisch mit dem Protein SipC von Salmonellen (Salmonellen-SipC-Protein) reagieren

Ausgehend von dem Salmonellen-Stamm Salmonella enterica subsp. enterica serovar enteritidis wurde bakterielle genomische DNA isoliert und mit Hilfe von SipC-spezifischen Primern das SipC-Gen amplifiziert und kloniert.

Anhand einer anschliessenden Sequenzanalyse und eines Datenbankabgleichs wurde die Bestätigung geliefert, daß es sich bei dem klonierten DNA-Fragment um den für SipCkodierenden Leserahmen handelt. Das errechnete Molekulargewicht des abgeleiteten SipC-Proteins beträgt etwa 42 kDa.

Nach Umklonierung in den Expressionsvektor pET30a (Novagen) - oder einen anderen geeigneten Expressionsvektor - wurde das Antigen in Fusion mit einem Histidin-Tag rekombinant in E. coli hergestellt und anschließend affinitätschromatographisch aufgereinigt.

Die Reinheit des auf diese Weise gewonnenen Antigens war ausreichend um es zur Immunisierung von Kaninchen und Mäusen einzusetzen.

Mehrere Mäuse wurden mit dem vorstehend beschriebenen rekombinanten Salmonellen-Antigen SipC grundimmunisiert und nachfolgend zwei Boosterimmunisierungen unterworfen. Die isolierten Milzzellen (Antikörperproduzierende B-Lymphozyten) dieser Tiere wurden anschliessend mit Myelomzellen (einer Tumorzelllinie, welche unbegrenzt wächst, selbst aber keinen Antikörper mehr produziert) fusioniert und die erhaltenen Hybridome selektioniert und anschliessend kloniert. Einzelne Klone wurden dann immunologisch auf die Produktion spezifischer monoklonaler Antikörper gegen das zur Immunisierung verwendete rekombinante SipC-Antigen gescreent. Bei diesem Screening wurden die beiden monoklonalen Antikörper ISB2 und V1H7 erhalten, die von den Zellen der bei der DSMZ-Deutsche Sammlung von Mikroorganismen und Zellkulturen GmbH, Deutschland, am 01. Juni 2006 unter den Hinterlegungsnummern DSM ACC2790 (I5B2) und DSM ACC2789 (V1H7) hinterlegten Hybridoma-Zellkulturen erzeugt werden.

Für Vergleichsversuche wurde mit demselben vorstehend beschriebenen rekombinanten Salmonellen-Antigen SipC ein polyklonaler Antikörper hergestellt. Hierfür wurden Kaninchen mit dem Antigen zunächst grundimmunisiert und nachfolgend mehreren Boosterimmunisierungen unterworfen. Aus dem Blut der Kaninchen wurde schließlich ein polyklonales Antiserum erhalten.

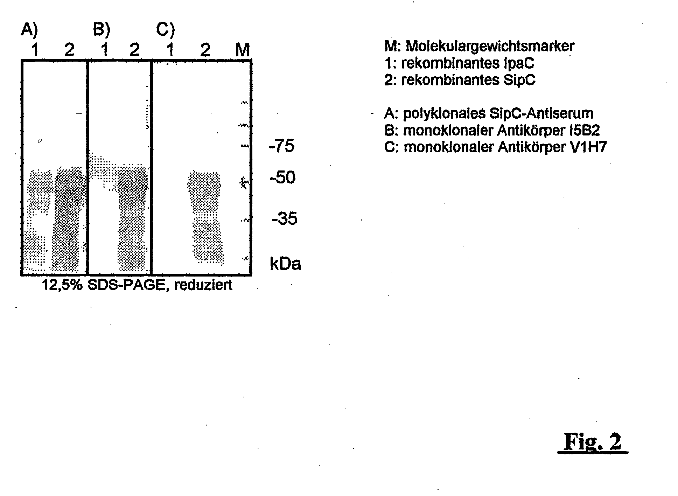

Die Spezifität der gewonnenen monoklonalen und polyklonale Antikörper wurde im Western Blot getestet. Figur 2 zeigt Western Blot-Streifen, welche mit dem vorstehend beschriebenen polyklonalen SipC-Antiserum (A), dem monoklonalen Antikörper I5B2 (B) und dem monoklonalen Antikörper V1H7 (C) entwickelt wurden. Aufgetragen ist jeweils rekombinantes IpaC (Spur 1) sowie rekombinantes SipC (Spur 2). Bei dem IpaC-Antigen handelt es sich um ein sequenzhomologes Protein aus dem Bakterium Shigella flexneri. Im Gegensatz zu dem polyklonalen Antiserum, welches auch mit dem homologen Protein IpaC von Shigellen kreuzreagiert (Figur 2A), sind die monoklonalen Antikörper I5B2 und V1H7 spezifisch für das Salmonellenantigen SipC (Fig. 2B und C).

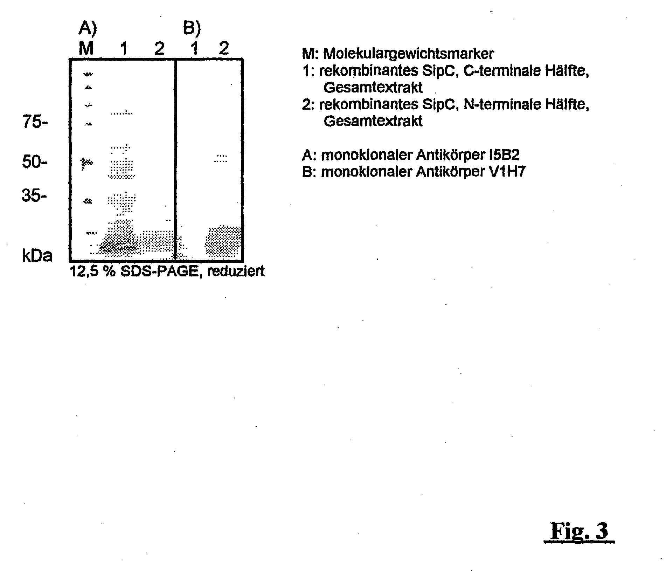

Um zu untersuchen, ob die monoklonalen Antikörper I5B2 und V1H7 gegen verschiedene Epitope des SipC-Antigens gerichtet sind, wurden Teilfragmente des Salmonellen-Antigens rekombinant hergestellt und im Western Blot mit den monoklonalen Antikörpern detektiert. Figur 3 zeigt entsprechend entwickelte Western Blot-Streifen, wobei in Spur 1 ein die C-terminale Hälfte von SipC (Aminosäuren 252-410) und in Spur 2 ein die N-terminale Region von SipC (Aminosäuren 1-200) enthaltender bakterieller Gesamtextrakt aufgetragen ist. Es zeigt sich, dass die beiden monoklonalen Antikörper eindeutig gegen verschiedenen SipC-Epitope gerichtet sind, da der mAk I5B2 (Figur 3A) mit dem C-terminalen SipC-Fragment, der mAk V1H7 (Figur 3B) dagegen mit der N-terminalen Hälfte von SipC reagiert.

### Beispiel 2: Nachweis von diversen Salmonellen -Serovaren mittels poyklonalem Antiserum gegen rekombinant hergestelltes Salmonellen-SipC-Protein

Jeweils 3 ml LB-Medium ("LB"= Luria Broth) wurden mit einer einzelnen Bakterienkolonie der in Tabelle 1, Spalte 1 aufgelisteten Salmonella-Stämme beimpft und für 16 Stunden bei 37°C unter leichtem Schütteln inkubiert.

Anschließend wurden die Bakterien mittels Zentrifugation abgetrennt und der erhaltene Überstand mit einer Endkonzentration von 10% TCA auf Eis gefällt.

Nach einem Waschschritt mit eiskaltem Aceton (-20°C) wurde das Proteinpellet getrocknet, in SDS-Gel-Probenpuffer resuspendiert, aufgekocht und auf einem 12,5% SDS-Gel gelelektrophoretisch aufgetrennt.

Die Übertragung der Proteine auf eine PVDF-Membran erfolgte mittels semi-dry Blotting. Die Membran wurde nach Absättigung aller unspezifischen Bindungsstellen zunächst mit dem gemäß Beispiel 1 hergestellten polyklonalen SipC-Antikörper/Antiserum (Verdünnung 1:10.000) und nachfolgend mit einem gegen Kaninchen-Immunoglobulin gerichteten und alkalische-Phosphatase-markierten Sekundärantikörper entwickelt.

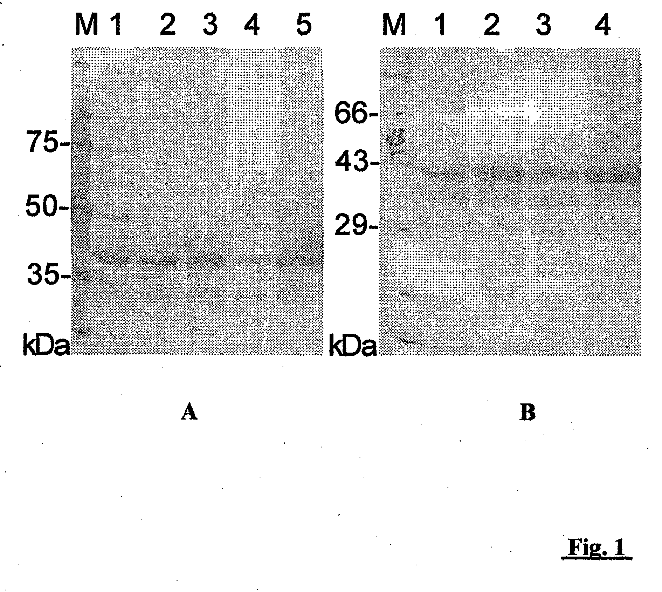

Figur 1 zeigt das Ergebnis dieses Western Blots: Bei allen eingesetzten Salmonellen-Stämmen ist im Molekulargewichtsbereich 42 kDa, das heißt in dem errechneten Molekulargewichtsbereich des Proteins SipC, eine deutliche Hauptbande detektierbar.

Das bedeutet, daß das SipC-Protein von allen in Tabelle 1 genannten Salmonellen-Stämmen gebildet und in den Überstand sezerniert wird. Zusätzlich zeigt dieser Versuch, daß das SipC-Protein/-Gen serovarübergreifend hoch konserviert ist.

Diese überraschenden Erkenntnisse weisen das SipC-Protein als besonders geeignet für einen Serovar-übergreifenden immunologischen Salmonellen-Nachweis aus.

### Beispiel 3: Immunologischer Nachweis des SipC-Antigens in Kulturüberständen verschiedener klinischer Salmonellen-Isolate mittels ELISA und Western Blot

Zum immunologischen Nachweis des SipC-Antigens aus Kulturüberständen von wurden jeweils 5ml LB-Medium mit einer Bakterienkolonie beimpft und über Nacht bei 37°C unter leichtem Schütteln inkubiert.

Die dabei verwendeten und gemäss Kauffmann-White-Schema charakterisierten Salmonellen-Stämme sind in Tabelle 2 aufgelistet.

Die Bakterienkulturen wurden am nächsten morgen bei 4000 x g (15 min, 4°C) abzentrifugiert, die gewonnenen Überstände durch eine 0,22µm-Membran sterilfiltriert und anschliessend in die diagostischen Assays (ELISA, Western Blot) eingesetzt.

### A) ELISA-Verfahren zum SipC-Nachweis aus bakteriellen Kulturüberständen

Mit dem in Beispiel 1 beschriebenen monoklonalen Antikörper I5B2 wurden ELISA-Platten beschichtet (1µg/well) und nach Blockierung unspezifischer Bindungsstellen mit einer 20%igen Gelantine-Lösung jeweils 150 µl des gewonnenen bakteriellen Zellkulturüberstandes pro Well pipettiert. Als Positivkontrolle diente eine definierte Menge rekombinantes SipC-Protein (5 ng/Well), als Negativkontrolle wurde steriles Nährmedium sowie gleichbehandelter Kulturüberstand anderer darmpathogener Bakterienspezies *(Escherichia coli, Yersinia enterocolitica)* eingesetzt. Die Platten wurden für 1h bei 37°C inkubiert und anschliessend mit TBST (Tris buffered Saline Tween 20) gewaschen. Zur Detektion des gebundenen Antigens wurde das in Beispiel 1 beschriebene polyklonale SipC-Antiserum nach ProteinG-Reinigung (Konz. 4 mg/ml) in einer Verdünnung von 1:50.000 eingesetzt. Nach einem erneuten Waschschritt mit TBST schloss sich eine einstündige Inkubation mit einem alkalische Phosphatase-markierten Anti-Kaninchen-Konjugat an, welches anschliessend durch erneutes Waschen mit TBST entfernt wurde. Durch Zugabe und die nachfolgende, von der antikörpergekoppelten alkalische Phosphatase katalysierten Umsetzung des Substrats 4-Nitrophenyl-Phosphat (gelöst in alkalische Phosphatase Puffer, 1 mg/ml) wurde spezifisch gebundenes Antikörperkonjugat colorimetrisch sichtbar gemacht und nach 30 Minuten Reaktionszeit die optische Dichte bei 405 nm bestimmt.

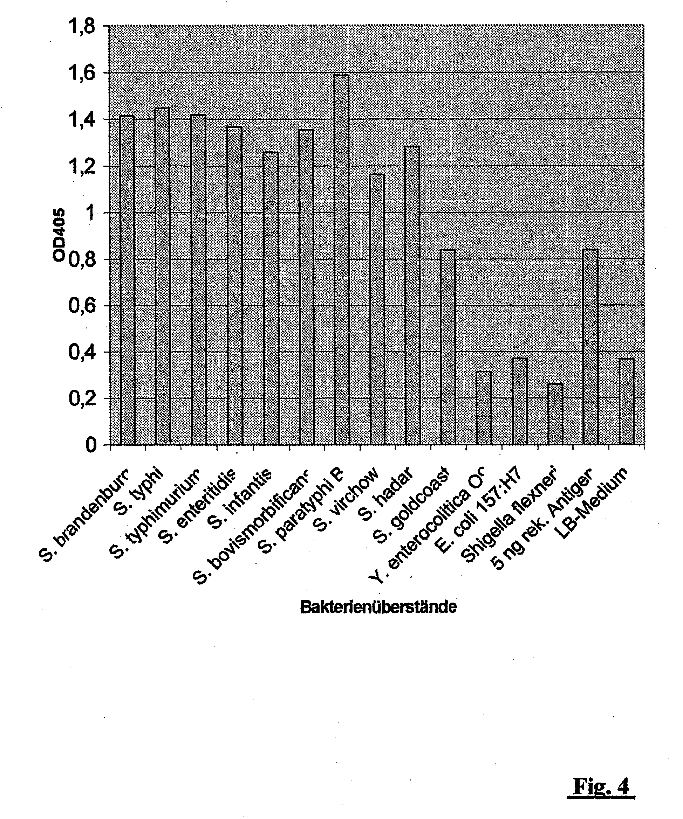

Wie Figur 4 entnommen werden kann, zeigen alle getesteten Salmonellen-Kulturüberstände eine positive Reaktion im ELISA, wogegen die Extinktion gleichbehandelter Überstande von anderen bakteriellen darmpathogenen Bakterien (E. coli, Yersinia enterocolitica, Shigella flexneri) der Extinktion des sterilen Nährmediums entspricht.

### B) Western Blot-Verfahren zum SipC-Nachweis in Bakterienkulturüberständen:

15µl der gewonnenen bakteriellen Zellkulturüberstände wurden über ein 10%iges SDS-Polyacrylamid-Gel aufgetrennt und anschliessend mittels dem Semi-Dry-Blotting Verfahren auf eine PVDF-Membran übertragen. Unspezifische Bindungsstellen wurden mit 3% BSA (Bovines Serumalbumin) in TBST blockiert. Anschliessend erfolgte die Inkubation mit dem SipC-spezifischen monoklonalen Antikörper I5B2 (350µg/ml nach ProteinG-Reinigung, 1:100 verdünnt in TBST). Nach drei Waschschritten mit TBST wurde die Membran für eine weitere Stunde mit einem alkalische Phosphatase-Anti-Maus-Konjugat inkubiert und danach erneut mehrfach mit TBST gewaschen. Die Entwicklung erfolgte durch die Zugabe von Substrat in Form BCIP (5-Bromo-4-Chloro-3-indolylphosphat-p-Toluidiniumsalz, 50 mg/ml in 100% Dimethylformamid, 1:300 in alkalische Phosphatase-Puffer) und NBT (50 mg/ml Nitro Blau Tetrazolium in 70% Dimethylformamid, 1:150 in alkalische Phosphatase-Puffer) für 5 Minuten bei Raumtemperatur.

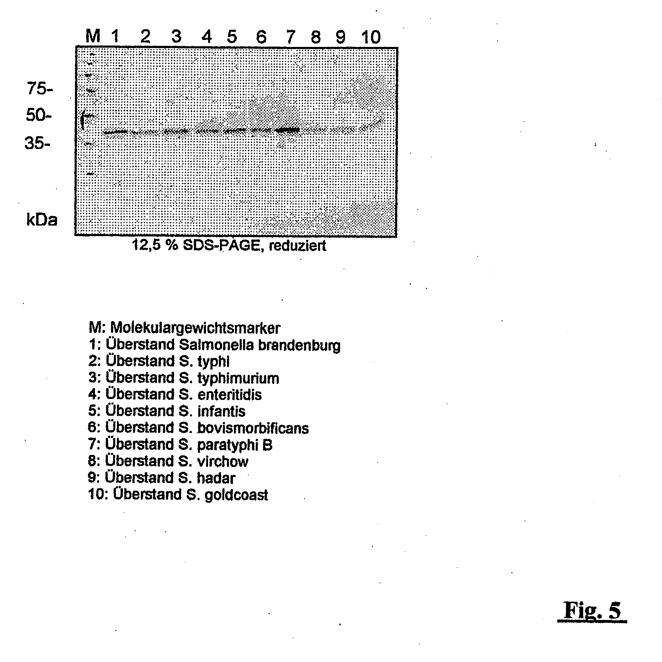

Die Signale des in Figur 5 abgebildeten Western Blots korrelieren sehr gut mit den zuvor geschilderten ELISA-Daten. Auch hier zeigen alle getesteten Salmonellen-Kulturüberstände eine positive Reaktion mit dem monoklonalen Antikörper I5B2 im zu erwartenden Grössenbereich von 42 kDa. Ferner existiert eine gute Korrelation zwischen der Intensität der Western Blot-Signale und den gemessenen Extinktionen im ELISA So zeigt zum Beispiel Überstand von S. paratyphi B in beiden Fällen die höchste Reaktivität.

### Beispiel 4: Zeitabhängiger immunologischer Nachweis des SipC-Antigens in Kulturüberständen von Salmonella enteritidis und S. typhimurium

Wie in Beispiel 3 gezeigt, stellt der SipC-ELISA ein hochspezifisches und sensitives Testverfahren zum Nachweis unterschiedlicher Salmonellen-Stämme dar. Mit dem vorliegende Beispiel 4 wird beispielhaft gezeigt, daß dieser SipC-ELISA außerdem die vorteilhafte Eigenschaft aufweist, dass er zu einem sehr frühen Zeitpunkt einer mutmaßlichen Salmonelleninfaktion durchgeführt (angewendet) werden kann, weil das SipC-Protein bereits zu einem sehr frühen Zeitpunkt gebildet wird und effizient nachweisbar ist.

Zur Demonstration des zeitlichen Verlaufs der SipC-Expression wurde wie folgt verfahren: 20 ml LB-Medium wurden mit jeweils einer Bakterienkolonie beimpft und bei 37°C unter Schütteln inkubiert. Während neun Stunden wurde stündlich eine Probe entnommen, abzentrifugiert und der sterilfiltrierte Überstand mit Hilfe des in Beispiel 3(A) beschriebenen ELISA-Verfahrens auf die Anwesenheit von SipC-Protein untersucht.

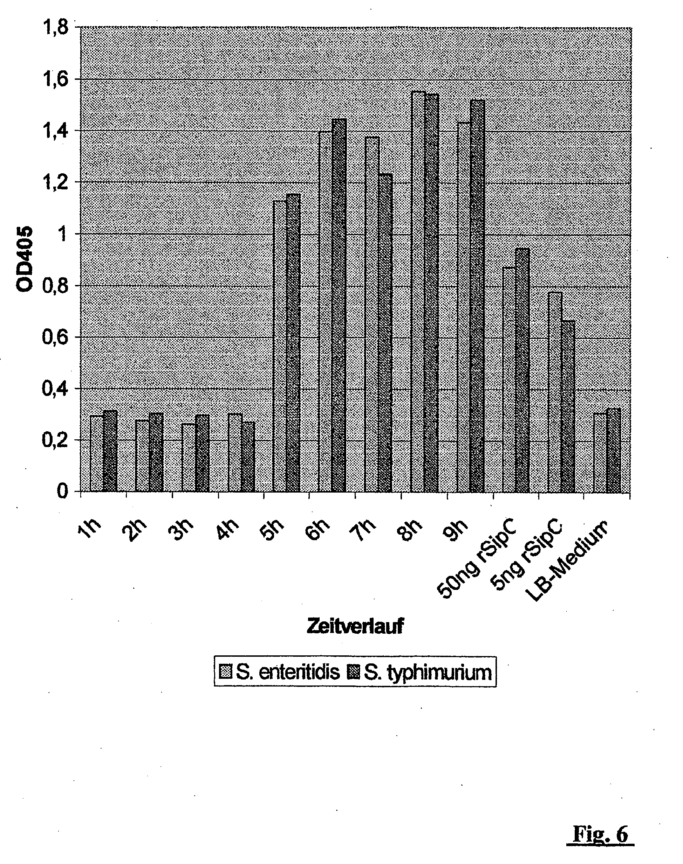

Die Ergnisse dieses Veruschs sind in Figur 6 dargestellt. Aus dieser Figur 6 ist ersichtlich, dass das SipC-Protein erstmalig bereits fünf Stunden nach Kulturstart im Überstand spezifisch nachgewiesen werden kann. Während der folgenden vier Stunden steigt die Menge an SipC-Protein nicht messbar weiter an.
Überraschenderweise werden die Proteine des TypIII-Sekretionssystems also bereits zu einem sehr frühen Zeitpunkt gebildet.

Aufgrund alledem ist der SipC-ELISA hervorragend zum schnellen Nachweis von Salmonellen geeignet.

### Beispiel 5: Immunologischer Nachweis von SipC-Antikörpern im ELISA

Um zu testen, ob in infizierten Tieren ein Nachweis von Anti-SipC Antikörpern aus dem Blut möglich ist, wurde untersucht wie effizient SipC die Ausbildung von Antikörpern induziert.

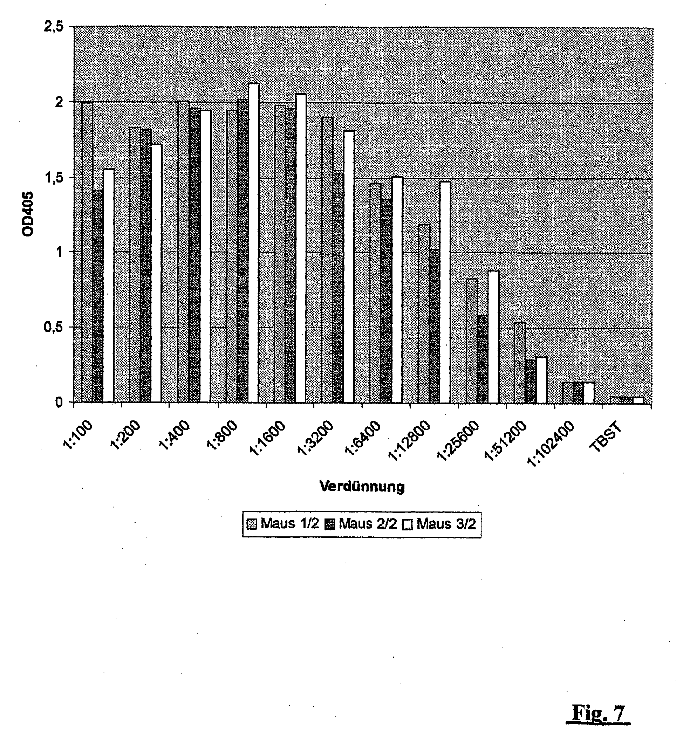

Dazu wurde drei Mäusen rekombinantes SipC- Protein appliziert. Anschließend wurde den Mäusen eine geringe Menge Blut entnommen und nachfolgend in einem spezifischen ELISA gegen SipC-Antikörper austitriert (vgl Figur 7). Hierfür wurde jeweils 1µg rekombinantes SipC-Protein pro Well an eine ELISA-Platte angelagert, anschliessend unspezifische Bindungsstellen mit einer 20%igen Gelantine-Lösung blockiert und schließlich mit den in Figur 7 aufgeführten Verdünnungsstufen der Seren inkubiert. Die Detektion von spezifisch gebundenen Antikörpern erfolgte mithilfe eines alkalische Phosphatase-markierten Anti-Maus-Konjugat mit dem Fachmann geläufigen Methoden. Nach Zugabe von 4-Nitrophenyl-Phosphat als Substrat für die antikörpergekoppelte alkalische Phosphatase wurde anhand der Substratumsetzung das spezifisch gebundene Antikörperkonjugat colorimetrisch sichtbar gemacht und nach 30 Minuten Reaktionszeit die optische Dichte (als Maß für die Menge an umgesetztem Substrat bzw. gebundenem Antikörperkonjugat) bei 405 nm bestimmt.

In Figur 7 ist erkennen, daß das Serum aller drei Mäuse dabei sehr weit (bis ca. 1:50.000) ausverdünnbar war. Die Stärke der Immunantwort gegen das Protein war in allen drei Mäusen nahezu identisch.

Diese Ergebnisse zeigen, daß es sich bei SipC um ein hochimmunogenes Protein (= Protein mit hoher Antigenität) handelt, das in allen infizierten Tieren eine deutliche Immunantwort, nämlich eine effiziente und spezifische Antikörper-Antwort gegen das SipC Protein, auslöst.

Mit Hilfe des ELISAs-zur Detektion von spezfischen SipC-Antikörpern wird hiermit erstmals die Bestimmung des Anti-SipC-Titers im Serum infizierter Menschen oder Tiere möglich. Auch die Detektion von Anti-SipC-Antikörpern im Fleischsaft von Nutztieren kann damit erfolgen. Der ELISA zur Detektion von SipC-Antikörpern stellt somit ein spezifisches und effizientes Testverfahren zur Identifizierung von bestehenden oder vergangenen Salmonellen-Infektionen dar.

### Literatur:

Komoriya K. et al.: Flagellar proteins and type III-exported virulence factors are the predominant proteins secreted onto the culture media of Salmonella typhimurium. Mol. Microbiol. (1999), 34(4), 767-779

Hueck C.J. et al.: Salmonella typhimurium secreted invasion determinants are homologous to Shigella Ipa proteins. Mol. Microbiol. (1995), 18(3), 479-490

Hueck C.J.: Type III protein secretion systems in bacterial pathogens of animals and plants. Microbiol. Mol. Biol. Rev. (1998), 379-433

Daefler, S.: Type III secretion by Salmonella typhimurium does not require contact with a eukaryotic host. Mol. Microbiol. (1999), 31(1), 45-51

Medizinische Mikrobiologie und Infektiologie. H. Hahn, D. Falke, S.H.E. Kaufmann, U. Ullmann (Hrsg.), Springer Verlag Berlin Heidelberg New York, Dritte Auflage 1999.

Kaniga K. et al.: Homologs of the Shigella IpaB and IpaC invasins are required for Salmonella typhimurium entry into cultured epithelial cells. J. Bacteriol. (1995); 177(14), 3965-3971.

Thomson, G.T. et al.: Serological testing for reactive arthritis. Clin. Invest. Med. (1994), 17(3), 212-217.

Van der Heijden, H.M.: First international ring trial of ELISAs for Salmonella antibody detection in swine. Berl. Munch. Tierärztl. Wochenschr. (2001), 114 (9-10), 389-392.

Ausubel, F.M. et al.: Current Protocols in Molecular Biology (2003), John Wiley and Sons. Inc.

**Tabelle 1: Auflistung der in Beispiel 2 verwendeten Salmonellen-Serovare und ihrer Antigenformel (Kauffmann-White-Schema)**

| **Salmonellen-Stamm** | **O-Antigen** | **Geißel-Antigen H** | |
|---|---|---|---|
| | | **Phase 1** | **Phase 2** |
| *S. hadar* | 6, 8 | z₁₀ | e, n, x |
| *S. brandenburg* | 1, 4, 12, 27 | l, v | e, n, z₁₅ |
| *S. goldcoast* | 6, 8 | r | l, w |
| *S. senftenberg* | 1, 3, 19 | g, [s], t | - |
| *S. virchow* | 6, 7 | r | 1,2 |
| *S. typhimurium* | 1, 4, [5], 12 | i | 1,2 |
| *S. enteritidis* | 1, 9, 12 | [f], g, m, [p] | [1, 7] |
| *S. infantis* | 6, 7, 14 | r | 1,5 |
| *S. bovis morbificans* | 6, 8 | r, [i] | 1, 5 |

**Tabelle 2: Auflistung der in Beispiel 4 verwendeten Salmonellen-Serovare und ihrer Antigenformel (Kauffmann-White-Schema)**

| **Salmonellen-Stamm** | **O-Antigen** | **Geißel-Antigen H** | |
|---|---|---|---|
| | | **Phase 1** | **Phase 2** |
| *S. hadar* | 6, 8 | z₁₀ | e, n, x |
| *S. brandenburg* | 1, 4, 12, 27 | l, v | e, n, z₁₅ |
| *S. goldcoast* | 6, 8 | r | l, w |
| *S. virchow* | 6, 7 | r | 1,2 |
| *S. typhimurium* | 1, 4, [5], 12 | i | 1, 2 |
| *S. enteritidis* | 1, 9, 12 | [f], g, m, [p] | [1, 7] |
| *S. infantis* | 6, 7, 14 | r | 1, 5 |
| *S. bovis morbificans* | 6, 8 | r, [i] | 1,5 |
| *S. typhi* | 9, 12, [vi] | d | - |
| *S. pararyphi B* | 1,4, [5], 12 | [b] | [1,2] |

## Patentansprüche

1. Testsystem für den Nachweis von gegenwärtig und/oder in der Vergangenheit vorhandenen Salmonellen-Infektionen in einer Probe, **dadurch gekennzeichnet, dass** das Testsystem wenigstens einen monoklonalen Antikörper umfasst, der spezifisch mit dem Salmonellen-SipC-Protein reagiert, und der (a) von den Zellen der bei der DSMZ-Deutsche Sammlung von Mikroorganismen und Zellkulturen GmbH, Deutschland, am 01. Juni 2006 unter der Nummer DSM ACC2790 hinterlegten Hybridoma-Zellkultur mit der Laborbezeichung 15B2 erzeugt wird, oder der (b) von den Zellen der bei der DSMZ-Deutsche Sammlung von Mikroorganismen und Zellkulturen GmbH, Deutschland, am 01. Juni 2006 unter der Nummer DSM ACC2789 hinterlegten Hybridoma-Zellkultur mit der Laborbezeichung V1H7 erzeugt wird.

2. Testsystem nach Anspruch 1, **dadurch gekennzeichnet, daß** das Testsystem ein ELISA ist.

## Claims

1. Test system for detection of a currently existing and/or in the past existing Salmonella infection in a sample, **characterized in that** the test system comprises at least one monoclonal antibody that reacts specifically with the Salmonella SipC protein and that (a) is produced by cells of the hybridoma cell culture with lab code I5B2 deposited on June 1, 2006 under the number DSM ACC2790 with the DSMZ German Collection of Microorganisms and Cell Cultures GmbH, Germany, or that (b) is produced by cells of the hybridoma cell culture with lab code V1H7 deposited on June 1, 2006 under the number DSM ACC2789 with the DSMZ German Collection of Microorganisms and Cell Cultures GmbH, Germany.

2. Test system according to claim 1, charcterized in that the testsystem is an ELISA.

## Revendications

1. Système de test pour la détection d'infections à la salmonelle présentes actuellement ou ayant été présentes dans le passé dans un échantillon, **caractérisé en ce que** le système de test comprend au moins un anticorps monoclonal qui réagit de façon spécifique avec la protéine SipC de salmonella, et (a) qui est obtenu par les cellules de la culture de cellules hybridomes déposée le 1^{er} juin 2006 sous le numéro DSM ACC2790 auprès de DSMZ-Deutsche Sammlung von Mikroorganismen und Zellkulturen GmbH, Allemagne, ayant le numéro de laboratoire 15B2, ou (b) qui est obtenu par les cellules de la culture de cellules hybridomes déposée le 1^{er} juin 2006 sous le numéro DSM ACC2789 auprès de DSMZ-Deutsche Sammlung von Mikroorganismen und Zellkulturen GmbH, Allemagne, ayant le numéro de laboratoire V1H7.

2. Système de test selon la revendication 1, **caractérisé en ce que** le système de test est un ELISA.
